# EUROPEAN PATENT APPLICATION

(11) **EP 1 094 036 A2**
(43) Date of publication of application: **25.04.2001**
(21) Application number: 00309218.6
(22) Date of filing: 19.10.2000
(51) Int. Cl.: C02F 1/36, A61L 2/025

(54) **Fluid treatment by filtration and vibration**

(30) Priority: 19.10.1999 GB 9924589
(71) Applicant: Snowball, Malcolm Robert, Epping, Essex CM16 6TW (GB)
(72) Inventor: Snowball, Malcolm Robert, Epping, Essex CM16 6TW (GB)
(74) Representative: Evans, Huw David Duncan

(57) **Abstract**

A fluid treatment apparatus comprises a fluid inlet 12 and outlet 13, a granular filter media 15 arranged to filter fluid flowing from the inlet 12 to the outlet 13 and a device 14 arranged to vibrate or agitate the media as fluid flows through the media between the inlet 12 and outlet 13.

Vibrating or agitating the granulated media in this way produces a grinding effect which serves to kill any micro-organisms caught in the filter by rupturing their cell walls.

## Description

The present invention relates to fluid treatment and more particularly but not solely to the treatment of potable water to remove parasites and harmful protozoa, such as cryptosporidium and giardia.

It is generally accepted that most raw water sources around the world contain harmful protozoa such as cryptosporidium and giardia. These parasites are extremely resistant to conventional methods of disinfection and now constitute a severe health hazard to the public in general.

It was thought that conventional sand filtration would provide an adequate barrier to guard against these parasites. This has been proved not to be the case, by the many outbreaks recorded around the world in recent years.

Technologies such as chlorine and biocides are ineffective against these micro-organisms. Ozone and micro-filtration have proved to be very expensive with the latter producing a bio-hazardous waste. UV light has proved effective in some applications but its efficiency is badly affected by particulate in the water to be treated.

An object of this invention is to provide a fluid treatment device which is highly efficient at killing parasites such as cryptosporidium and giardia, which does not produce a bio-hazardous waste, which is economic, energy efficient, substantially self-cleaning and unaffected by particulate in the water.

In accordance with this invention there is provided a fluid treatment apparatus, comprising a fluid inlet and outlet, a granular filter media arranged to filter fluid flowing from the inlet to the outlet and means for vibrating or agitating the media as fluid flows through the media between the inlet and outlet.

It has been found that when a granulated media is vibrated or agitated, and in particular with ultra sonic vibration it produces a multi-collision grinding effect on a micro scale, which is extremely efficient at killing micro-organisms. Thus, if a liquid containing parasites such as cryptosporidium or giardia passes through the vibrating media the parasites are killed due to the media colliding with the parasites, crushing them and rupturing of their cell walls. The process is highly efficient and repeatable.

Also, in accordance with this invention, there is provided a method of treating fluid comprising passing the fluid through a granular filter media and vibrating or agitating the media whilst fluid continues to flow through the filter media.

Embodiments of this invention will now be described by way of examples only and with reference to the accompanying drawings, in which:
FIGURE 1 is a sectional view through an embodiment of fluid treatment apparatus in accordance with this invention;
FIGURE 2 is a sectional view through a second embodiment of fluid treatment apparatus in accordance with this invention;
FIGURE 3 is a sectional view through a third embodiment of fluid treatment apparatus in accordance with this invention; and
FIGURE 4 is a sectional view through a fourth embodiment of fluid treatment apparatus in accordance with this invention.

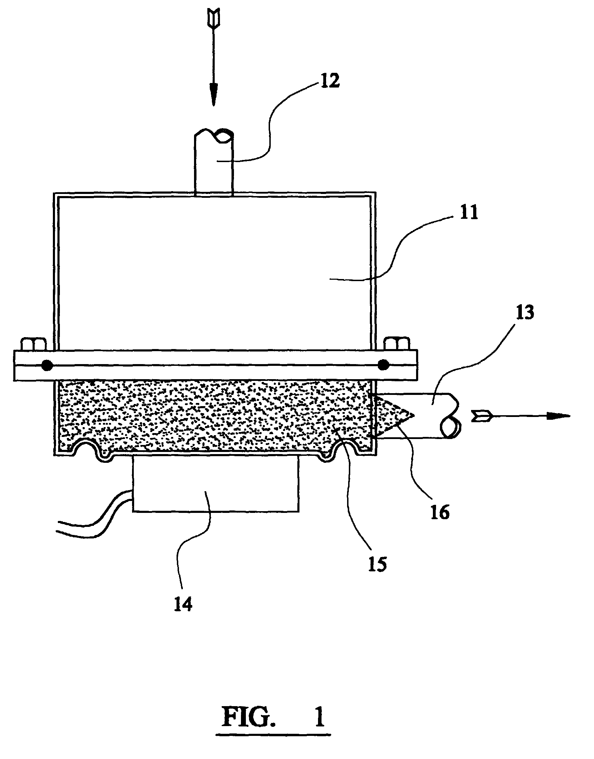

Referring to Figures 1 of the drawings, there is shown a fluid treatment apparatus comprising a watertight chamber 11 sealed at both ends and having an inlet port 12 and an outlet port 13. The chamber can be split in two for maintenance via flanges 17 and water tight seal 19. Positioned on and around the base of the chamber is a layer of granulated media 15. Outside the chamber and attached to the base of the chamber, which is formed to act like a diaphragm, is a vibrator 14.

Preferably the vibrator is an ultra sonic vibrator, which when activated vigorously vibrates the layer of granulated media. Placed in front of the outlet port is a mesh 16, which retains the media inside the chamber.

Referring to Figure 1 of the drawings, assuming that the ultra sonic vibrator 14 is 'on' and the layer of granulated media 15 is vibrating. The liquid to be treated flows into the chamber via inlet port 12 through the vibrating media 15, through the retaining mesh 16 and out of the chamber 11 via the outlet port 13. Any parasites such as cryptosporidium and giardia are killed when passing through the vibrating media due to mechanical abrasion, collisions and the micro grinding effect.

The continuous vibration keeps the media clean and free from debris build up with particulate either being ground up and passed through the system or if hard granulated, adding to the media. The continuous vibration also keeps the pressure drop across the system at a minimum by maintaining the media loose packed.

In the case of drinking water the system is not effected by chlorine therefor the chlorine residual can be maintained in the water ready to protect the mains.

The system is modular in construction with several units able to be manifolded in parallel to increase the system throughput.

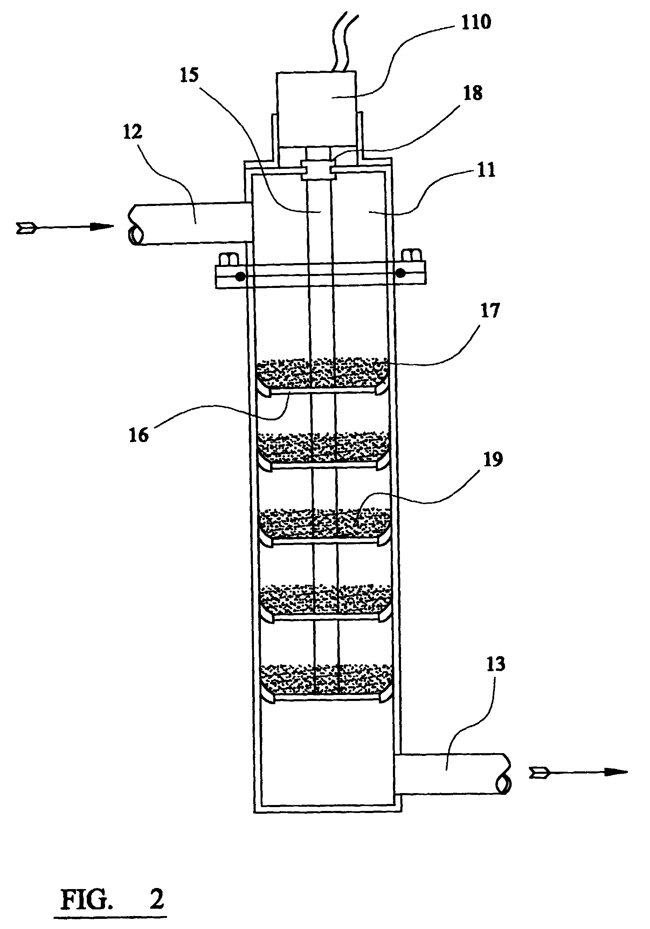

In a second embodiment shown in Figure 2 a cylindrical watertight chamber 11 sealed at both ends have an inlet port 12 and an outlet port 13. The chamber can be split in two for maintenance via flanges 111 and water tight seal 112. Passing through one end of the chamber 11 via seal 18 is a shaft 15, which runs through the chamber, parallel to the walls of the chamber. Fixed to the shaft 15 is a Plurality of perforated disks 16, each disk has flexible seals 17, attached to their periphery. Part or all of the space between each perforated disk is filled with a layer of granulated media 19 which is maintained in place by the disk 16, flexible seal 17 and the wall of the chamber 11. On the outside of the chamber, fixed to the shaft 15, is a vibrator 110.

Preferably the vibrator is an ultra sonic vibrator, which when activated vigorously vibrates the layers of granulated media between the disks 16 via shaft 15.

The invention will now be described in detail with the aid of Figure 2. Assuming that the ultra sonic vibrator 14 is 'on', the shaft 15 vibrates causing the layers of granulated media 19 to vibrate. The liquid to be treated flows into the chamber via inlet port 12, through each of the perforated disks of vibrating media 16, and out of the chamber 11 via the outlet port 13.The flexible seals 17 make a watertight seal to the wall of the chamber so the liquid cannot bypass the media 19.

Any parasites such as cryptosporidium and giardia are killed when passing through the disks of vibrating media due to mechanical abrasion, collisions and the micro grinding effect previously described in embodiment 1.

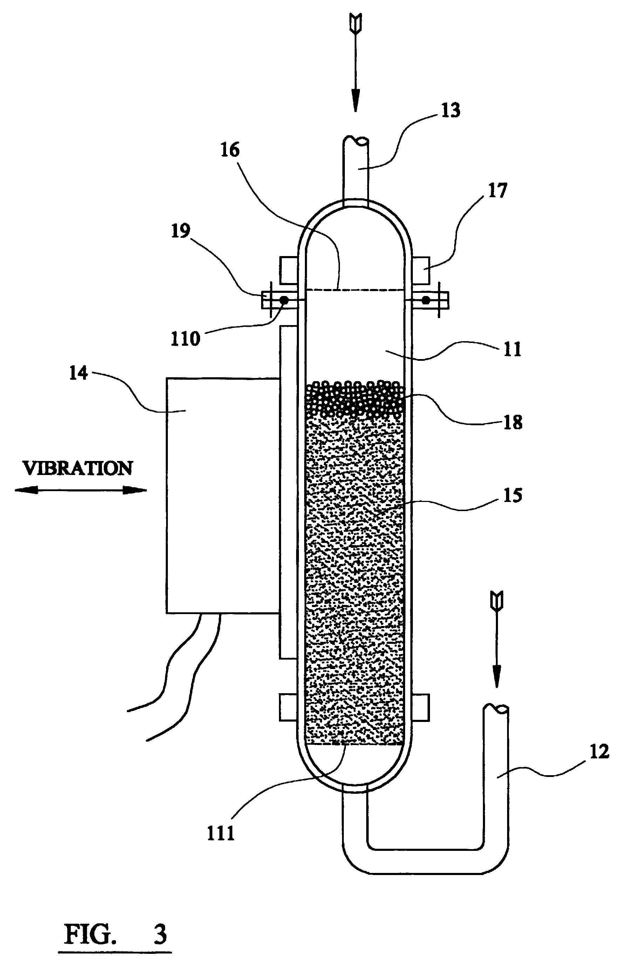

In a third embodiment shown in Figure 3 a cylindrical watertight chamber 11 sealed at both ends has an inlet port 12 and an outlet port 13. The chamber can be split in two for maintenance via flanges 19 and water tight seal 110. The chamber is supported at both ends by rubber mounts 17, which allows the cylindrical chamber some movement. Partially filling the chamber is a column of granulated media 15, on top of which is placed a layer of large particle size media 18. Outside the chamber and attached to the wall of the chamber is a vibrator 14.

Preferably the vibrator is an ultra sonic vibrator, which when activated vigorously vibrates the column of granulated media in the direction across the diameter of the chamber. Placed in front of the outlet and inlet ports are meshes 16 & 111, which together with the large size media 18 retains the granulated media inside the chamber.

The invention will now be described in detail with the aid of Figure 3. Assuming that the ultra sonic vibrator 14 is 'on' and the column of granulated media 15 is vibrating, the liquid to be treated flows into the chamber via inlet port 12, through mesh 111, through the column of vibrating media 15, through the retaining mesh 16 and out of the chamber 11 via the outlet port 13. Any parasites such as cryptosporidium and giardia are killed when passing through the vibrating media due to mechanical abrasion, collisions and the micro grinding effect previously described in embodiment 1.

In some circumstances it is desirable to have a disposable cartridge type system. For instance, a system suitable for domestic applications would need this facility.

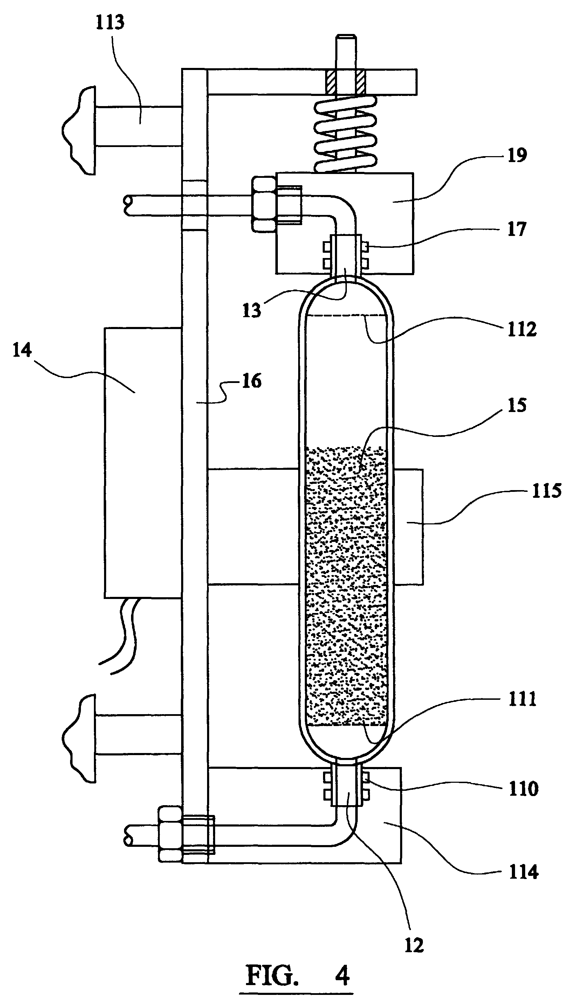

In a forth embodiment shown in Figure 4 a cylindrical watertight disposable cartridge 11 has an inlet port 12 and an outlet port 13. The cartridge 11 Fixes onto the 'L' shaped mounting plate 16 via mounting 114 and the spring-loaded connector 19 and support block 115. The plate 16 is resiliently mounted in a stationary position via resilient mounts 113. The cartridge is sealed to the inlet and outlet ports 12 & 13 in a watertight manner by 'O' ring seals 17 & 110. Partially filling the cartridge and supported by mesh 111 & 112, is a column of granulated media 15. Attached to the "L" shaped plate 16 is a vibrator 14.

Preferably the vibrator is an ultra sonic vibrator, which when activated vigorously vibrates the plate and hence the column of granulated media, in the direction across the diameter of the cartridge. The outlet port mesh 111 retains the granulated media inside the cartridge.

The invention will now be described in detail with the aid of Figure 4. Assuming that the ultra sonic vibrator 14 is 'on' and the cartridge 11 of granulated media 15 is vibrating via the vibrating 'L' shaped plate 16. The liquid to be treated flows into the cartridge 11 via inlet port 12, through the retaining mesh 111, through the column of vibrating media 15, through the retaining mesh 112 and out of the chamber 11 via the outlet port 13.

Any parasites such as cryptosporidium and giardia are killed when passing through the vibrating media due to mechanical abrasion; collisions and the micro grinding effect previously described in the first embodiment.

In alternative embodiments, the vibratory devices 14,110 may be replaced by agitators.

## Claims

1. A fluid treatment apparatus, comprising a fluid inlet and outlet, a granular filter media arranged to filter fluid flowing from the inlet to the outlet and means for vibrating or agitating the media as fluid flows through the media between the inlet and outlet.

2. A fluid treatment apparatus as claimed in claim 1, in which said means for vibrating or agitating the media is arranged to vibrate the media at an ultrasonic frequency.

3. A fluid treatment apparatus as claimed in claims 1 or 2, in which said means for vibrating or agitating the media is arranged to vibrate or agitate a wall which contains said media.

4. A fluid treatment apparatus as claimed in claims 1 or 2, in which the media is contained within a flow duct of the apparatus, said means for vibrating or agitating the media being arranged to vibrate or agitate the flow duct.

5. A fluid treatment apparatus as claimed in any preceding claim, comprising a plurality of series-connected stages each having a granular filter media arranged to filter fluid flowing from the inlet to the outlet and means for vibrating or agitating the media as fluid flows through the media between the inlet and outlet.

6. A method of treating fluid comprising passing the fluid to be treated through a granular filter media and vibrating or agitating the media whilst fluid continues to flow through the filter media.
